(19) Europäisches Patentamt
European Patent Office
Office européen des brevets

(11) **EP 4 613 201 A1**

(12) **EUROPEAN PATENT APPLICATION**

(43) Date of publication:
**10.09.2025 Bulletin 2025/37**

(21) Application number: **24305357.6**

(22) Date of filing: **07.03.2024**

(51) International Patent Classification (IPC):
***A61B 5/374*** (2021.01)   ***A61B 5/00*** (2006.01)

(52) Cooperative Patent Classification (CPC):
**A61B 5/374; A61B 5/7264;** A61B 5/7267;
A61B 2503/045

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicants:
- **Assistance Publique - Hôpitaux de Paris**
  **75012 Paris (FR)**
- **Sorbonne Université**
  **75006 Paris (FR)**
- **Institut National de la Santé et de la Recherche Médicale (INSERM)**
  **75013 Paris (FR)**

- **Institut du Cerveau et de la Moelle Épinière-ICM**
  **75013 Paris (FR)**
- **Centre National de la Recherche Scientifique**
  **75016 Paris (FR)**

(72) Inventors:
- **VERMERSCH, Anne-Isabelle**
  **75012 PARIS (FR)**
- **CHAVEZ, Mario**
  **75013 PARIS (FR)**
- **FIAMMANTE, Marc**
  **06800 CAGNES SUR MER (FR)**

(74) Representative: **Flesselles, Bruno F.G.**
  **BF IP**
  **36 rue Jean de la Fontaine**
  **75016 Paris (FR)**

(54) **METHOD FOR PROCESSING AN ELECTROENCEPHALOGRAM SIGNAL**

(57)    The invention pertains to a new computer-implemented method for processing an EEG signal, that makes it possible to extract information about cerebral damage in full-term babies, born in asphyxia context, for help in an indication for treatment of hypoxic-ischemic encephalopathy (HIE). The method comprises computing a power spectral density (PSD) of an EEG signal, applying a mathematical function to transform the PSD, processing the transformed values by data binning, determining durations of binned values, and creating a probability distribution of the durations of the binned values.

Figure 3

**Description**

The invention pertains to a new computer-implemented method for processing an EEG (electroencephalogram) signal, that makes it possible to extract objective information about cerebral damage in full-term neonates, born in asphyxia context, withing the first six hours of life after birth, confirming or not an indication for treatment of treatment of hypoxic-ischemic encephalopathy (HIE).

Perinatal asphyxia is an imprecise term that corresponds to a severe alteration of uteroplacental gas exchange, leading to metabolic acidosis (Antonucci et al., 2014). In this context, all organs can be affected, but the neonate's brain is particularly vulnerable. Brain damage due to perinatal asphyxia constitutes hypoxic-ischemic encephalopathy (HIE) (Volpe et al, 2012). Early therapeutic hypothermia (TH) is the reference neuroprotective treatment for neonatal HIE (Martinello et al., 2017). It consists in placing, within the first six hours of life, the child in hypothermia (whole body cooling) for 72 hours involves sedation and assisted ventilation of the child. The objective of TH is to reduce the severity of brain damage by preserving cerebral energy metabolism, thus making it possible to reduce the severity and extent of the cortical, thalamic and hippocampal lesions observed after a HIE (Colbourne et al, 2000 and Bona et al, 1998). Perinatal asphyxia cannot generally be prevented.

In contrast with the spontaneously favorable evolution of minor EHI, hypothermia is currently exclusively reserved for moderate and severe HIE where it significantly reduces the number of deaths and reduces neurological sequelae (Jacobs et al., 2007, Edwards et al., 2010). Although it has an established neuroprotective action, TH is a "heavy" treatment, requiring hospitalization at NICU, most often including deep sedation with intubation of the child. Furthermore, hypothermia has side effects, some of which can have severe consequences such as heart rhythm and coagulation disorders (Jannatdoost et al., 2013).

In the current practice of neonatal intensive care units (NICU), resuscitators face with a very short time window of 6 hours, within which they must decide to initiate invasive therapeutic hypothermia, while decision criteria are most often incomplete. Furthermore, the decision to start TH is often made without EEG criteria, which deprives the resuscitator of a precise functional evaluation of the cerebral electrical activity of the child. and therefore, of the certainty of the presence/absence of cerebral lesions.

Hypoxic-ischemic encephalopathy (HIE) defined by Sarnat and Sarnat (1976) calls on clinical criteria (e.g. level of consciousness, presence, or absence of reflexes in the newborn, etc.), biological (pH, lactate levels), and EEG markers (background rhythms and presence or not of critical discharge). The analysis of background EEG signals provide accurate prognostication in the two extremes of neonatal HIE grades. Whereas normal or mildly affected EEG is associated with a good clinical outcome (Gray, 1993), moderate or severe HIE may have a poor clinical outcome if the TH is not applied on time (Lawn et al., 2005 and Dixon et al., 2002). The identification of neonates suitable for TH must be therefore done rapidly after birth, and it is a challenge in clinical care neonatal of asphyxiated neonates.

Although continuous EEG is a widely available non-invasive bed-side test, it requires specific technical skills for its recording and interpretation to recognize neonatal brain function abnormalities in full-term infants born in a context of asphyxia. Among other characteristics, pediatric neurologists often consider features of EEG activity such as amplitude, frequency, continuity and sleep-wake cycling (Korotchikova et all, 2009). The identification of low voltage background activity, inactive phase or burst-suppression, very long inter-bursts intervals (IBIs), and theta-delta inversion are some elements suggesting the existence of a brain injury. Nevertheless, the lack of access to continuous EEG recording and analysis, is crucial to identify a HIE grade requiring the initiation of hypothermia (Dilena et al., 2021). In France, for instance, only 7% of NICUs have recently reported having 24/7 access to continuous EEG (Chaton et al., 2023).

In contrast to continuous EEG captures much of the rich neonatal cortical dynamics, the amplitude integrated EEG (aEEG) offers a simplified, compressed trace derived from standard EEG (al Naqeeb N, et al. 1999; Hellstrom-Westas L). This compressed representation of EEG is a tool easier to access and requiring less expertise than continuous recordings analysis, which can also be used over longer periods of time (Shellhaas et al., 2007). Nevertheless, aEEG has also technical and interpretation pitfalls, and evidence has shown that it may underestimate HIE severity (Evans et al., 2010). Indeed, although aEEG can accurately detect normal or severely injured neonates, there is a lack of sensitivity for mild to moderate HIE severity (Marics et al., 2013). There is therefore a real need for an automatic system of continuous EEG interpretation, that could robustly characterize the large variety of background patterns to identify the progression of an encephalopathy in neonates with HIE.

Several algorithms have been proposed to identify, from the EEG analysis, full-term neonates with HIE candidate to a therapeutic hypothermia. Many studies have proposed many reliable EEG features, including parameters from the time, frequency or information theory domain to quantify EEG in full-term neonates with HIE (L6fgren et al., 2006; Korotchikova et al., 2011; Lacan et al., 2021). Some studies have proposed the interburst intervals as a parameter to quantify the evolution of encephalopathy in neonates with HIE (Thordstein et al., 2004; Löfhede et al., 2008; 2010). Nonstationary statistics, based on time-frequency decompositions (Stevenson et al., 2013; Raurale et al., 2021) or the analysis of long-range fluctuations (Matic et al., 2015), have been proved to be a useful tool to quantify the continuity of background EEG, providing thus a marker of the encephalopathy severity in neonates. Quantification of spatiotemporal dynamics of raw or parametrized EEG signals has also been proposed as discriminating features to identify HIE grades (Stevenson et al., 2013; Matic et al., 2014; Wang et al, 2022). In all these approaches, different feature sets are estimated from EEGs before combining with machine learning algorithms (Ahmed et al., 2016) or, more recently, with deep-learning methods (Raurale et al., 2021; Moghadam et al., 2022), to provide an automated grading system of HIE.

Previous studies have shown that delta (0.5 - 4 Hz) and theta (4 Hz - 8 Hz) oscillations are predominant in newborn cortical activities (Tsuchida et al., 2013).

There is therefore a real need for an automatic system of continuous EEG interpretation, that could robustly characterize the large variety of background patterns to identify the progression of an encephalopathy.

The objective of the invention is to propose a simple real-time system, based on EEG signal analysis before H6 of life, to identify any sign of brain injury during the evolution over the course of EEG monitoring, facilitating thus the indication or not for a therapeutic hypothermia for full-term neonates, born in a context of asphyxia. The invention is based on the dynamics of slow EEG oscillations (summarized by the levels of delta power with respect to their duration), which can accurately identify the group of neonates requiring hypothermia treatment. It was shown that these features are statistically robust providing thus the basis for a simple and reliable decision support tool for neonatologists in the NICU.

The invention proposes a new method for processing an EEG signal, which can be used before H6 of life of a newborn, to identify any sign of brain injury, thereby facilitating the indication or not for a therapeutic hypothermia for full-term neonates, born in a context of asphyxia.

According to one embodiment, it is possible to obtain specific information from the spectral features of slow EEG oscillations (the levels of delta power and theta/delta ratio with respect to their duration) which can be compared to reference data, to accurately identify a HIE grade requiring hypothermia treatment. These features are statistically robust providing thus the basis for a simple and reliable decision method and process for neonatologists in the NICU.

As summarized, the method uses

- Computation of power spectral densities of at least one frequency band of the EEG signal, for a multiplicity of consecutive time windows
- Transforming the obtained values, through the use of a mathematical function
- Performing data binning to classify the various transformed values in various classes (bins)
- Creating a distribution of the durations of the binned values
- And optionally displaying a graph of the distribution.

In one embodiment, the invention pertains to a computer implemented method for processing an Electroencephalogram (EEG) signal, comprising

a) Computing Power Spectral Density (PSD) of at least one frequency band of the EEG signal, for each time window of a multiplicity of consecutive time windows, to obtain computed PSD values,

b) Transforming the computed PSD values by applying a mathematical function to the computed PSD values to obtain transformed values

c) Processing the values obtained in b) by data binning, by replacing each transformed value, which fall into a given interval by a value representative of that interval, to obtained binned values,

d) Sequentially determining durations of the binned values, wherein a duration of a given binned value is obtained from the number of consecutive time windows for the given binned value,

e) Creating a probability distribution of the durations of the binned values.

Each computed PSD value obtained in a) is thus associated with a given time window, namely the one from which it has been computed.

The method is performed *ex vivo.* It is performed on an EEG that has been independently recorded.

The EEG signal has preferably been obtained from frontal, parietal, central or occipital electrodes, or from a montage of these electrodes. In particular, the EEG signal is obtained from frontal electrodes or from a frontal-temporal montage. When performed on newborns, the EEG may have been acquired using the Incereb Neon Neonatal Electrodes commercialized by Lifelines Neuro Company, LLC (Louisville, Kentucky, USA), which presents frontal, parietal, central and occipital electrodes.

In the first step, an EEG signal is split into consecutive time windows, spanning the whole EEG signal. The durations of each time window are identical.

It is desired when the total duration of the EEG signal is at least 15 minutes. In another embodiment, the total duration of the EEG signal is at least 20 minutes. It is preferred when the total duration is at least 30 minutes. A duration of the EEG between 30 and 40 minutes is adequate for performing the method.

In one embodiment, the duration of each time window duration is comprised between 0.5 and 2 seconds, preferably between 0.5 and 1.5 seconds. A duration of 1 second for each time window is adequate.

When a duration of 1 second for each time window is used, and the duration of the analyzed EEG is 30 minutes, a total number of 1800 time windows are obtained.

For each time window of the multiplicity of time windows, the total spectral power or the mean power spectral density (PSD) is computed for at least one frequency band of the EEG signal. In the framework of the invention, and as explained below, the term PSD may be used to designated either the mean power spectral density or the total spectral power.

It is preferred when the frequency band of the EEG signal is selected from the delta waves, alpha waves, beta waves, theta waves frequency bands and association of these bands.

It is reminded that the alpha wave band is the 8-12 Hz band (12.0 Hz not included), the beta wave band is the 12-30 Hz band (30.0 Hz not included), the delta wave band is the 0.5-4 Hz band (4 Hz not included), and the theta wave band is the 4-8 Hz (8.0 Hz not included) band.

In one embodiment, the total spectral power (see below) is computed from the delta wave band.

In one embodiment, the total spectral power is computed from the theta wave band.

In one embodiment, the total spectral power is computed from the alpha wave band.

In one embodiment, the total spectral power is computed from the beta wave band.

In one embodiment, the total spectral power is computed from the theta wave and the delta wave bands. In this embodiment, two total spectral power values are obtained.

In one embodiment, the total spectral power is computed from the 0.5-8 wave band. In this embodiment, one value is obtained.

In one embodiment, the total spectral power is computed from the 0.5-13 Hz wave band. In this embodiment, one value is obtained.

In one embodiment, the total spectral power is computed from the alpha wave and the delta wave bands. In this embodiment, two values are obtained.

In one embodiment, the total spectral power is computed from the alpha wave, the theta wave and the delta wave bands. In this embodiment, three values are obtained.

It is reminded that, generally speaking, the power spectral density is a measure of the power distribution across different frequencies in an EEG signal. Although one of skill in the art is aware of several methods to calculate PSD from EEG data, a widely used method is the Welch's method.

The EEG signal would be processed, in particular by filtering the data to remove frequencies outside the range of interest, removing or interpolating bad channels, and detrending or normalizing the data. As the studied signal is obtained from a limited time window (an epoch), a window function, such as the Hamming, Hanning, or Blackman window, is applied to reduce spectral leakage and improve the spectral resolution of the PSD estimate.

The Fast Fourier Transform (FFT) can then be applied to each windowed epoch to estimate the power spectrum. The power spectrum is the magnitude squared of the complex FFT coefficients, and represents the distribution of power across different frequencies.

Other methods could be envisaged for analyzing EEG data besides FFT, such as wavelet analysis, Hilbert-Huang transform, or event-related spectral perturbation (ERSP) analysis, which involves calculating a baseline spectral power for a given frequency band (for instance, the baseline could be the values just before induction of anesthesia), and comparing this baseline to the spectral power during anesthesia.

When multiple electrodes are used to acquire the EEG data, the PSD calculated for each electrode can then be averaged to obtain the final PSD for each time window.

It is possible to compute the PSD (as envisaged in a)), in one time window, for each frequency (from 0.5 Hz to 30.0 Hz), with a 0.1 Hz increment. For a given band (such as the delta wave band), it is possible to obtain the total spectral power by summing up (integrating) the values for the this given band. Such total spectral power can be used for implementing the methods herein disclosed. It is also possible to obtain a mean power spectral density by dividing the calculated total spectral power by the width of the band (3.5 for the delta band, 4 for the theta band, 4 for the alpha band, 18 for the beta band, 7.5 for the delta+theta band...). Such mean power spectral density for the band can also be used in the methods herein disclosed. As indicated above and in the context of the specification, the term "PSD values" can be used to designate the "total spectral power values" or "mean spectral power density values" thereby calculated, and used in the methods.

The obtained PSD values obtained in a) present a great variability, between time windows, and generally cover a large range of values. In order to perform the data binning of c), it is thus of interest to reduce the variability and the range of these values. Applying mathematical functions to these values makes it possible to achieve this objective.

In order to reduce the range of the PSD values, it is first possible to apply a logarithm function to such. One can use the neperian logarithm (In or log) but using the decimal logarithm (Log or $Log_{10}$), obtained by dividing the neparian logarithm by ln(10) is advisable. One could also use the square root, the cubic root (or another root).

As an illustration, when applying the Log function to the PSD calculated from a given wave band, the obtained value would vary between 0 and 9 (the original PSD values extend from 0 to about $10^9$). This is particularly true when applying the Log function to the PSD calculated for the delta waves.

In another embodiment, PSD values are computed for delta waves frequencies and another for theta waves frequencies. It is possible to first calculate the Theta PSD value / Delta PSD value ratio for each time window, and then apply a mathematical function as disclosed above, notably a $Log_{10}$ function to such calculated ratio. In some embodiments, one can calculate, for each time window, a (Theta PSD + k) / Delta PSD ratio, wherein k is a number comprised between $10^3$ and $10^4$ and applying a $Log_{10}$ function to calculated ratio. Using such number (constant) comprised between $10^3$ and $10^4$ makes it possible to obtain an accuracy of about 88%.

The values obtained after application of the mathematical function thus transform the computed PSD values to transformed values.

Such transformed values can then be subject to data binning, *i.e.* original transformed values which fall into a given interval (a bin) are replaced by a value representative of that interval.

When using values obtained from $Log_{10}$ transformation of the PSD values, one can round the value, either to the integer, or to the first or second decimal. It is however preferred to round the value to the first decimal place. Indeed, this would provide around 100 bins when using $Log_{10}$ transformed values (which would range from 0 to 9 as indicated above). It is indeed preferred when the binning method provides between 50 and 200 bins or between 80 and 120 bins, around 100 bins being perfectly adequate. If the mathematical value used in b) is different than the $Log_{10}$ transformation (including when it is the identity transformation), it is possible to manually determine the bounds for each bin and to allocate a given value to each PSD comprised between given bounds.

One thus has a list of couples of consecutive time windows together with the binned value for each time window.

The following step consists in sequentially determining durations of the binned values, wherein a duration of a given binned value is the number of consecutive time windows for the given binned value.

For implementing this step, one shall use the following algorithm

- Take the first time window, and determine the binned (rounded) value for this first time window
- Create an entry in a database, an entry in the database comprising a couple of values, the first value being the binned value, and the second value being 1 (one)
- Repeat the following steps, up to the last time window:

    ◦ Move to the next time window, and determine the binned (rounded) value for this next time window

        ▪ If the binned value for this next time window is identical to the one of the preceding time window, increment by one the second value of the last created entry in the database
        ▪ If the binned value for this next time window is different from the one of the preceding time window, create a new entry in the database, with the first value being the binned value of this next time, and the second value being one

As an example a binned values sequence of 1.1, 1.1, 2, 3.5, 3.5, 3.5, 3.5, 3.2, 3.0, 1.1, 1.1,... gives (value,duration) sequence (1.1,2), (2,1), (3.5,4), (3.2,1), (3.0,1), (1.1,2),... after processing.

The output of this implementation is a sequence of (binned values; number of consecutive time windows for which the binned value is identical, herein designated as the duration of the binned values). Such data can be stored in an appropriate table.

Such (value, duration) sequences can be used to create a value, duration, count pivot table (array) to obtain the count of occurrences of a given (value,duration) pair. Such pivot table can be normalized to obtain a probability distribution of the couples (value,duration), so that the total sum of probabilities over the distribution probability is 1.0

It is also possible to smooth the probability distribution by applying a gaussian kernel such as a (5,5) gaussian kernel (kernel density smoothing), to fill in missing values in the original probability distribution. Normalization after smoothing is to be performed to ensure that the resulting total distribution probability is indeed 1.0.

It is reminded that smoothing with a Gaussian kernel is a technique commonly used in signal processing and data analysis to reduce noise and emphasize trends in data. The process involves convolving the data with a Gaussian function, also known as a Gaussian kernel or Gaussian filter. This convolution replaces each data point with a weighted average of its neighboring points, with the weights determined by the Gaussian function. The width of the Gaussian function, known as the standard deviation, determines the extent of smoothing: a larger standard deviation results in more smoothing, while a smaller standard deviation provides more weight to the central value.

The coefficient to use for the Gaussian kernel can be automatically determined by any method available in the art. In particular, it is possible to call for a function such as the "getGaussianKernel" function from the OpenCV function library (opencv.org/), which returns Gaussian filter coefficients.

On can set up k at 5 (5x5 kernel), with a sigma of 1.1 (calculated according to the formula sigma = 0.3*((ksize-1) *0.5 - 1) + 0.8).

The kernel can thus be represented by the following array:

[0.00390625, 0.015625 ,0.0234375,0.015625 , 0.00390625],
[0.015625 , 0.0625 , 0.09375 , 0.0625 , 0.015625 ],
[0.0234375 , 0.09375 , 0.140625 , 0.09375 , 0.0234375 ],
[0.015625 , 0.0625 , 0.09375 , 0.0625 , 0.015625 ],
[0.00390625, 0.015625 , 0.0234375 , 0.015625 , 0.00390625]

As indicated, one could set-up a different sigma value, to provide more weight to points closer to the center, but a sigma between 1 and 1.2 is adequate for performing this smoothing.

One should also note that smoothing would provide values at some locations (delta power / duration) of the pivot table where no probability was initially present. However, some locations may remain empty.

Hence, it is also possible to perform at least another smoothing (generally no more than one other smoothing) to reduce the number of "empty" locations. For performing this second smoothing, one can use a 3x3 Gaussian kernel, or another 5x5 Gaussian kernel. It is advisable to use a sigma value lower than the sigma value used for the first smoothing. When using a 5x5 kernel for performing such second smoothing, a sigma value between 0.5 and 0.8 is adequate.

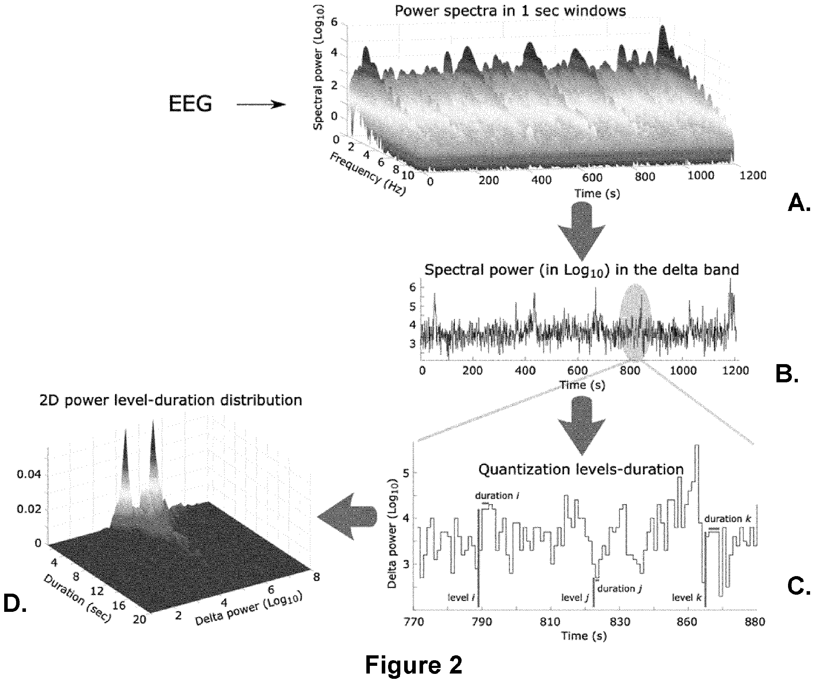

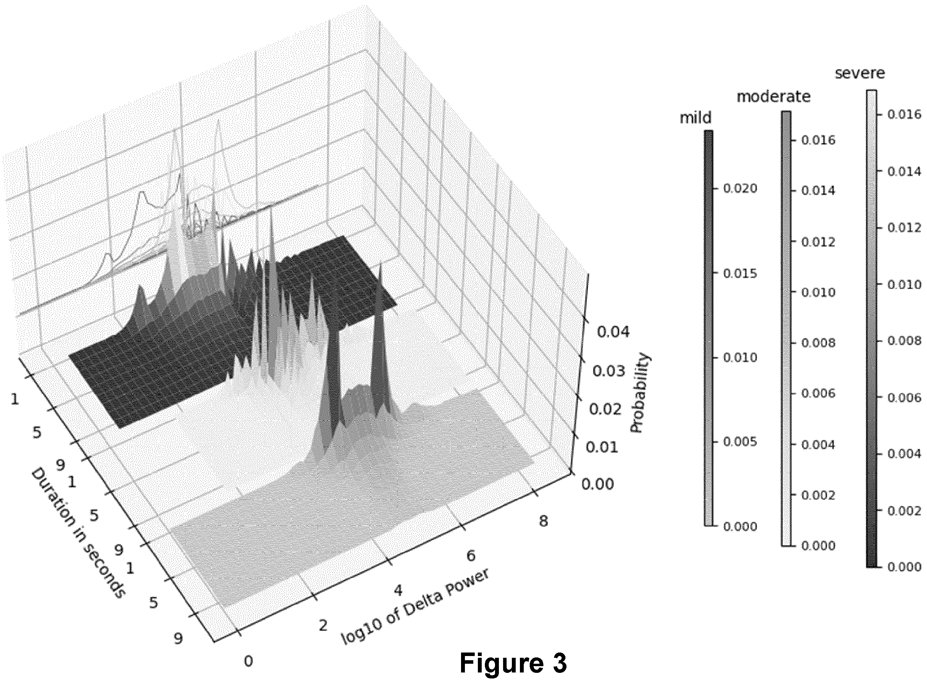

It is then possible to display or visualize the distribution by plotting the probability distribution on a graph with the duration on the x axis, the binned values on the y axis and the probability on the z axis. An example of such graphically displayed distribution can be seen on Figure 3 or Figure 2.D.

The method above thus transforms an EEG signal and makes it possible to obtain further information from it. It can notably be used for grading hypoxic-ischemic encephalopathy (HIE) in an infant. By comparing the distribution as calculated from the EEG obtained from the infant to reference distributions (representing EEG distribution for infants with no, low or high risk of HIE), it is possible to assign a class to the infant (no risk of HIE, low risk of HIE, high risk of HIE) and to perform the appropriate acts to avoid occurrence of side effects.

The invention thus also relates to a computer-implemented method for grading hypoxic-ischemic encephalopathy (HIE) in an infant, comprising

a. performing the method as described above with the EEG signal obtained from the infant, to obtain the probability distribution of the duration of binned values for the infant obtained from delta waves PSD or from the delta wave PSD / theta wave PSD ratio, and

b. calculating the (statistical) distance of the probability distribution to at least two reference distributions (one reference distribution representing a normal (non HIE) class (or grade) and another reference distribution representing a pathologic (HIE) class (or grade)).

Such method makes it possible to obtain a grade of hypoxic-ischemic encephalopathy in the infant.

One can compare the tested distribution to only two reference distributions (normal (absence of HIE) / pathologic (presence of HIE)).

In other embodiments, the tested distribution is compared to three reference distributions representative of three clinical grades (normal (absence of HIE) / mild HIE / severe HIE).

Another step of d. grading the hypoxic-ischemic encephalopathy (HIE) in the infant by assigning the probability distribution of the duration of binned values for the infant to the class of the reference distribution for which the distance is the lowest, or which present the least outliers with such infant's probability distribution.

It is also possible to provide a hypothermia recommendation if the grade falls in a "presence of HIE" category.

The reference distribution can be a probability distribution that was previously obtained from the EEG of an infant that has no brain injury (normal or mildly abnormal HIE grade). Alternatively, the reference distribution can be a probability distribution that was previously obtained from the EEG of an infant that has mild HIE (moderately abnormal), or severe HIE (severely abnormal or inactive EEG). Three classes are herein described, but it may be possible to use more classes (or less classes such as normal/pathologic). However, it is believed that two or three classes are an adequate number, as they provide the necessary information for the physician to decide the physician whether to start a treatment of the infant.

In one embodiment, multiple reference distributions are used in b., and wherein multiple distances are calculated, wherein each reference distribution corresponds to a clinical HIE grade, and wherein the infant is affected to the clinical grade for which the distance is the lowest. It is indeed preferable to compare the probability of the infant's generated distribution to one reference for each grade to make sure that the appropriate grade is assigned for the infant's clinical status.

In some embodiments, the reference distribution is the mean of multiple individual distributions obtained from EEG of multiple infants (preferably smoothed at least once with a 5x5 Gaussian kernel as indicated above, and optionally another time with a 3x3 or 5x5 other Gaussian kernel, if the number of "empty" locations (i.e. a location where no positive probability is present) is considered too high, which could generally not be the case if the number of individual distributions is high enough). Such reference is obtained by calculating, for a given (binned value;duration) couple, the mean of the probabilities for this (binned value;duration) couple from the multiple individual distributions, of the same class or grade.

It is thus of interest to have databases of normal and pathologic EEG of infantes to be able to obtain the reference distributions for each of the classes.

The (statistical) distance between the test probability distribution (obtained from the infant) and the reference probability distribution can be calculated according to any method known in the art. One can use a distance selected from the Bhattacharyya, Partial Curve Mapping, Area method, Discrete Frechet distance, Curve Length, Dynamic Time Warping, Mean absolute error, Mean squared error, Kullback-Leibler, and Jensen Shannon distances.

It is preferred to use the Euclidian distance between the two probability distributions, which is the square root of the sum of the squared differences of the probabilities for each point (duration/binned value) of the pivot table.

When the distance of the infant probability distribution is calculated for multiple reference probability distribution, one can use the following rules:

a. the lowest distance is the lowest calculated distance from the reference probability distributions for which there are no outliers

b. In case there are outliers for all reference distributions, the lowest distance is set as being the one to the reference probability distribution for which there is the lowest total probability for all outliers

wherein an outlier is a point that is present in the probability distribution of the duration of binned values for the infant and that is absent in the reference probability distribution. When comparing the tested EEG distribution to the reference distribution, the outlier probability is the sum of all probabilities from the tested EEG distribution at each point where the reference distribution is null.

In presence of at least one outlier, it is considered that the distance cannot be calculated and it is then considered to be infinite.

One can note that, due to the potential presence of outliers, the distance of the probability distribution of the duration of binned values for the infant to the reference probability distribution is not necessarily the same than the distance of the reference probability distribution to the probability distribution of the duration of binned values for the infant.

Indeed, it is possible that some points are present in the infant's generated probability distribution and absent in the reference probability distribution (presence of outliers), which would prevent calculation of the distance, as indicated above, whereas all points of the reference probability distribution have a counterpart in the infant's generated probability distribution, thus allowing calculation of a distance.

The smoothing with the gaussian kernel, as disclosed above, is thus of interest to reduce the number of points where no probability is found, and hence reduce the number of outliers. Such smoothing is thus performed for both the reference distributions and the infant's distribution. However, even with such smoothing, it may not be possible to allocate a value for some points.

Such points for which no probability can be assigned in the reference distribution, but for which a probability is present in the test distribution (the one obtained from the infant's EEG) are thus called outliers.

In summary, when calculating the distance, the first step would be to determine the number of outliers between the test distribution and the reference distributions.

If there are no outliers with all reference distributions, the test distribution is considered to be in the class of the reference distribution for which the distance is the smallest.

If there is an outlier with at leats one distribution, but no outliers with at least one other distribution, the distance is calculated between the test distribution and the reference distributions for which there are no outliers. The test distribution is considered to be in the class of the reference distribution for which the distance is the smallest (presence of at least one outlier in one reference distribution disqualifies this reference distribution, when there is no outlier in other reference distribution(s)).

If there are between the test distribution and all reference distributions, this indicates that the test distribution presents points that are not found in any of the references. This test distribution is considered to be in the class of the one for which there are the fewest outliers.

The method herein disclosed is preferably performed with EEG obtained from an infant within the first six hours of life after birth. It is advisable to perform the method when birth of the infant was accompanied with potential hypoxia. In particular, the method is preferably performed for infants with a gestational age > 36 weeks and a birth weight > 1,800 g. Potential hypoxia is to be presumed in the presence of at least one of the following features: (a) birth asphyxia with clinical suspicious features (e.g. fetal distress, umbilical cord prolapse); (b) Apgar score ≤ 5 at 5 or 10 minutes after birth; (c) pH < 7 in umbilical cord or any blood sample (arterial, venous or capillary) within 60 minutes of birth; (d) continued need for resuscitation, including endotracheal or mask ventilation, at 10 minutes after birth; or (e) lactates > 11 mmol/l in umbilical cord or any blood sample (arterial, venous or capillary) within 60 minutes of birth.

By providing a grade of the risk of hypoxic-ischemic encephalopathy, it is possible to provide a treatment to an infant found at risk (i.e. for which the distance of the probability distribution to the normal reference probability distribution is higher than the one to another reference probability distribution). Such treatment is preferably maintaining the infant at a temperature of 33°C for three days. In particular, maintaining core body temperature between 33°C and 34°C for 72 hours, followed by a period of rewarming of 6 to 12 hours, is envisaged.

## DESCRIPTION OF THE FIGURES

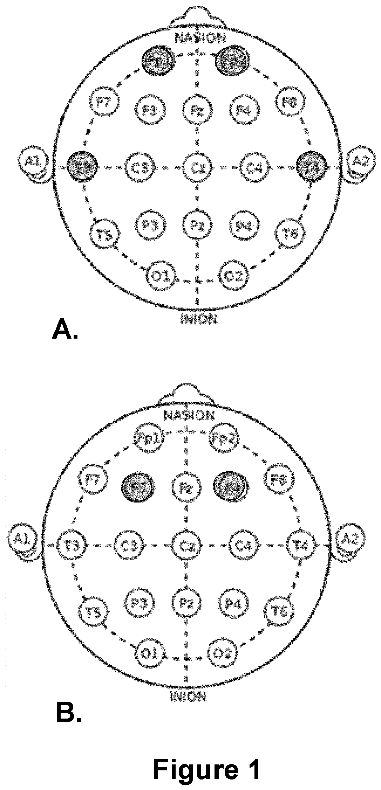

Figure 1: position of the preferred electrodes: Frontal-Temporal Fp1-T3, Fp2-T4 (A); Temporal F3, F4 (B)
Figure 2: Overview of the proposed feature extraction for each EEG channel. A. The Power Spectral Density is extracted from the EEG, and the $Log_{10}$ function is applied thereto. B. The PSD specific for the delta wave band is extracted. C. The values of the PSD are rounded to the first decimal (binned), and the sequences of durations are determined. D. The probability of couples (duration; binned PSD values) are plotted for graphical representation.
Figure 3: Delta Wave Power duration probability distribution references per Grade on all reference EEGs. Joint level-duration probability densities of delta power for the three grades of HIE. The normalized densities correspond to the average densities obtained from all mild, moderate, and severe cases.
Figure 4: Theta/Delta ratio duration probability distribution references per Grade.

## EXAMPLES

### Example 1. Abstract

**Objective:** Indication of therapeutic hypothermia needs an accurate identification of brain injury in the early neonatal period. Here, it is aimed to provide a simple hypothermia decision-making tool for the term neonates with hypoxic-ischemic encephalopathy (HIE) based on features of conventional electroencephalogram (EEG) taken less than 6 hours from birth.

**Methods:** EEG recordings from one hundred full-term babies with HIE were included in the study. Each EEG recording was graded by pediatric neurologists for HIE severity. Amplitude of each EEG segment was analyzed in the slow frequency bands. Temporal evolution of spectral power in delta (0.5 - 4 Hz) frequency band was used to characterize each HIE grade. For each grade of abnormality, level and duration (number of consecutive segments above a given level) probability densities for power of delta oscillations were estimated. This study was registered on the clinicaltrials.gov site (NCT05114070).

**Results:** These 2D representation of EEG dynamics can identify mild HIE group from those of requiring hypothermia. The discrimination system yielded an accuracy, recall, positive predictive value (precision), negative predictive value, false alarm ratio and F1-score of 98%, 99%, 99%, 0.94%, 0.06 and 99%, respectively. These results provided an accurate discrimination of mild versus moderate or severe HIE, and only one mild case was erroneously detected as relevant for hypothermia.

**Conclusions:** Quantized probability densities of slow spectral features (delta power) from early EEG (withing 6 hours of birth) revealed significant differences in slow spectral dynamics between infants with mild HIE grades and those relevant for hypothermia.

**Significance:** Conventional EEG segments can be represented by simple and interpretable biomarkers that can constitute a visual and efficient clinical decision support tool for physicians to identify full-term neonates with HIE candidates to a therapeutic hypothermia (TH).

Example 2. Methods

### 2.1. Dataset

The 104 neonates included in this retrospective study were part of a larger cohort of more than 600 term neonates monitored between 2005 and 2020, at the NICU of the Armand Trousseau Hospital (Paris, France). Following the French Society of Neonatology, the inclusion criteria were (Saliba et al, 2010): gestational age > 36 weeks and a birth weight > 1,800 g; postnatal age <H6; and presenting an HIE (mild, moderate or severe) with at least one of the following features: (a) birth asphyxia and with clinical suspicious features (e.g. fetal distress, umbilical cord prolapse); (b) Apgar score $\leq$ 5 at 5 minutes; and (c) pH < 7, and/or lactates > 11 mmol/l in cord blood or in the first hour of life.

The parents of patients who satisfied the inclusion criteria were informed by a written document of the anonymized use of data, and non-opposition consent was obtained. The study was declared to the French National Commission on Informatics and Liberty (agreement reference CER-2021-023), and it had the approval from the local Ethics Committee of Sorbonne University. This study was registered on the clinicaltrials.gov site (NCT05114070).

## 2.2. EEG recordings

EEG signals were recorded with a Deltamed (2005-2016) and a Nihon-Khoden (2016-2020) systems. EEG recording from three patients were excluded from the analysis because of technical format problems. To monitor the evolution of a possible encephalopathy, all recordings continuous video EEG recordings were obtained during the first six hours of birth after perinatal asphyxia.

EEG signals were obtained with Deltamed (N=27) and Nohon-Khoden (N=77) recording systems, sampled at 256 Hz and 500 Hz, respectively. Although scalp electrodes were positioned according to the international 10-20 system, only signals from electrodes Fp1, Fp2, T3 and T4 were considered in this study. In both recording systems, the reference electrode was placed on the midline between Fz and Cz. During acquisition, EEG signals were filtered using high and low-pass filters at 0.19 Hz and 70 Hz. Electrode impedance was always maintained below 10 kW during the whole recordings. Physiological measures of heart activity (ECG), respiration and SpO2 were simultaneously recorded for some patients, but these signals were not used in this study. In all analyses, EEG signals were segmented into 1s non-overlapping epochs. For each EEG recording, artefact periods were visually identified, and the corresponding segments were removed from the analysis. Segments with artefacts caused by large motion interferences, or bad electrode-tissue contacts were thus removed. EEG recordings from 4 patients were discarded from the study as files were corrupted (n=3) or data were strongly contaminated by artifacts during the whole recording (n=1).

For each patient, the degree of hypoxic-ischemic encephalopathy was evaluated according to Sarnat and Sarnat classification (Sarnat et al, 1976) within the first six hours after birth. Data were collected so that three HIE grades could be included: 17 of the 100 newborns included in the study were labelled as mild or with minor encephalopathy (continuous background with mild voltage depression [30-50 mV]); 17 as moderate (discontinuous activity with IBIs < 10s, clear asynchrony), and 66 as severe (IBIs of 10-60 s, severe background attenuation [<30 mV]) HIE. The demographics of the study is summarized in Table 1.

Table 1. Demographic characteristics of study group

| Demographic data (median value [min - max])] | | |
|---|---|---|
| Total | | 100 |
| Gender (M/F) | | 60/40 |
| Gestational age at birth (weeks) | | 39+3 [34+6 - 42+4] |
| Apgar 1 minute | | 2 [0 - 8] |
| Apgar 5 minutes | | 6 [1 - 10] |
| Umbilical cord gas PH | | 7.05 [6.67 - 12] |
| Lactates mmole/l | | 10.8 [1.7 - 21] |
| Age at EEG recording (h) | | 5 [2 6] |
| Analyzed EEG recording time (minutes) | | 30.8 [5.8 - 56] |
| *HIE severity groups* | | |
| Not requiring hypothermia | Mild HIE | 17 |
| Candidate to hypothermia | Moderate HIE | 17 |
| | Severe HIE | 66 |

## 2.3. EEG processing

A general scheme of the method is illustrated in Figure 2. For all EEG recordings, bipolar channels Fp1-T3 and Fp2-T4 were constructed, and power spectral density was estimated in all 1-second segments using Welch periodogram method. All segments where the alpha (8-12 Hz) spectral power was above $10^5$ mV$^2$/Hz were considered as artefacts and removed.

Although aEEG was originally developed as a bedside monitor for adult intensive care, it is now a widely used method for continuous monitoring of brain activities in NICUs. This signal is generally obtained from the EEG by asymmetric bandpass filtering (to reduce the effects of artefacts), rectifying (to transform biphasic into monophasic waveforms), smoothing and compression by a semi-logarithmic amplitude compression, followed by a time compression such that maxima and minima of transformed signal in a time window are displayed. This reduction of the EEG's information and time compression make continuous monitoring and bedside interpretation possible for long recordings.

Here, a more general representation of EEG background dynamics was created, by quantizing power levels of EEG and measuring the durations that the spectral power stays at different levels. Here the EEG oscillations at the delta (0.5 - 4 Hz) frequency band were considered. For each EEG segment, the power of delta band oscillations were first quantified. For this feature, a logarithmic transformation ($Log_{10}$) was used, and the values were quantized to the first decimal. In this way, small changes remain visible, while an overloading due to large values is avoided.

Pivot tables were then created with the sequence of levels/duration, capturing the information about the quantized power level (or power ratio) changes across EEG segments. Tables were normalized resulting in a joint duration/level probability table. Finally, a Gaussian smoothing was applied to obtain 2D smooth probability densities.

*2.4. HIE grade severity classification*

The ability of level-duration distributions to discriminate between different HIE grades were first evaluated, using a simple nearest-neighbor classifier. To assess the classifier's performances, a leave-one- out cross-validation was considered: for each fold, one EEG recording was randomly assigned to the testing dataset, while the remaining recordings were used to train the algorithm. An average probability distribution of each grade is firstly estimated from EEG recordings of the training set. Then, the 2D level-duration representation of the test data is compared with the three reference distributions to identify the nearest category (grade). The corresponding grade of the nearest neighbor is finally allocated to the test data. To compare two probability distributions *Pi* and *Pj* on sample space $\xi$, the variational (or $L_1$) statistical distance was evaluated, simply defined as $\frac{1}{2} \sum_{x \in \zeta} \left| P_i(x) - P_j(x) \right|$ (Basseville, 1989).

The ability of the classifier was finally evaluated to distinguish mild HIE group from those requiring hypothermia (severe and moderate HIE merged in a single class). Classification performances were assessed by the accuracy (to measure how often the learning model correctly predicts the outcome), recall or sensitivity (to quantify how good the learning model is at finding all the positives), precision (also called positive predictive value, which quantifies the proportion of true positives to the amount of total positives that the model predicts), balanced accuracy (the average of sensitivity and specificity that accounts for both the positive and negative outcome classes, and thus for class imbalance), negative predictive value, false positive rate (false alarm ratio) and F1-score, which is a weighted average of the recall and precision.

**Example 3. Results**

*3. 1. Results on the presented dataset*

Figure 3 shows the densities corresponding to the fluctuations of delta power, for the mild, moderate and severe HIE categories. It can be noticed that these 2D representations, created for the EEG signals of each patient, show shape differences for all HIE grade categories that can be easily recognizable by humans. The detection of different EEG segments yielded a direct grade match for 81% of EEGs, specifically 53% severe, 16% mild, and 12% moderate. The remainder is an adjacent grade matching with only one mild graded as moderate, four moderate graded as severe, and thirteen severe misclassified as moderate. No mild was identified as severe or vice versa. The discrimination of mild HIE group from those infants requiring hypothermia (severe and moderate) yielded an accuracy, recall, positive predictive value (precision), balanced accuracy, negative predictive value, false alarm ratio and F1-score of 98%, 99%, 99%, 94%, 96%, 6% and 99%, respectively. Only one mild was misclassified as requiring hypothermia.

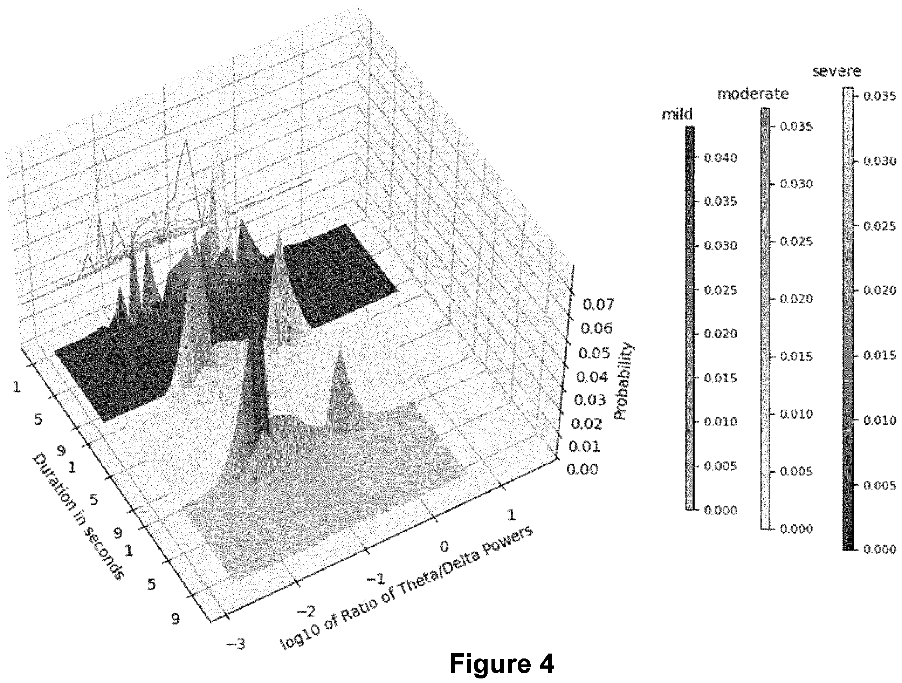

Figure 4 shows the densities corresponding to the fluctuation of the Theta / Delta ratio.

Although the combination of other spectral features could also be used as a marker of normality in neonatal EEG, the discrimination between EEG segments of the mild HIE group from those relevant for hypothermia yielded moderate performances. The theta/delta spectral ratio, for instance, provided an accuracy, recall, positive predictive value (precision), balanced accuracy, negative predictive value, false alarm ratio and F1-score of 80%, 96%, 79%, 82%, 70%, 55% and 87%, respectively.

*3.1. Results from a public EEG-HIE dataset*

For clarity and reproducibility purposes, the method was tested on a publicly available dataset made available by the Cork University Maternity Hospital, Ireland (O'Tool et al., 2023). This dataset consists of more than 100 multichannel EEG files, recorded at the NICU from 53 full-term newborns with a diagnosis of HIE. In the absence of pre-frontal electrodes, for the analysis of this dataset the signals recorded at the frontal regions (electrodes F3 and F4) were used. Each 1-hour EEG recording was graded for severity of background abnormalities. It is worthy to notice that contrary to the EEGs used in the initial database, that were obtained before 6 hours of age, the public dataset includes EEG recordings obtained for a prolonged period up to 100 hours after birth. Further, graduation of Cork data considers four HIE grades:

normal or mildly abnormal, moderately abnormal, severely abnormal, and inactive.

When applied to this dataset, the classification based on the delta power yielded a direct grade match for 77% of EEGs, the remainder was an adjacent grade matching with 9.6% of normal graded as moderately abnormal, whereas 1.9% of moderately abnormal were misclassed as severely abnormal, and only 0.95% of severe were erroneously matched as inactive. The discrimination of the normal HIE group (normal and mildly abnormal considered together) from those infants requiring hypothermia (severely abnormal and inactive merged as a single class) yielded an accuracy, recall, positive predictive value (precision), balanced accuracy, negative predictive value, false alarm ratio and F1-score of 94%, 86%, 86%, 96%, 91%, 3.6% and 86%, respectively.

## Example 4. Conclusion

The results are consistent with those of prior studies using quantitative EEG in full-term neonates, and support simple automated EEG analysis as an accessible, generalizable method for generating biomarkers of brain injury in neonates with HIE. It is worth noticing that the 2D level-duration probability distributions obtained for each EEG capture well some characteristics of different HIE grade categories that can be easily recognizable by clinicians: for the mild grade, the shape of the distribution allows a clear distinction between awake and sleep states. The two main pics reflect the distinctive patterns of high voltage and lower-voltage waveforms observed in the trace alternant activity during the quiet sleep, whereas the rich variability of EEG fluctuations of the awake state is captured in the bumpy central values. The decrease of EEG amplitude, characteristic of moderate HIE grade, can also be observed in the right shift of the density toward lower values of power. Finally, when compared with the level-duration distribution of the mild group, the 2D density associated to severe HIE grade clearly reflects the discontinuous and very low amplitude of EEGs.

The findings show that the spectral fluctuations of EEG background, captured by the 2D level-duration distributions of delta power, can reliably be used to discriminate mild versus moderate or severe HIE. Indeed, with the proposed approach, an accuracy of 98% was obtained, with only one mild case erroneously detected as relevant for hypothermia (1%). When applied to a public dataset with four HIE grades, the proposed system achieved an overall classification accuracy (considering the four grades) of 77%, with only 0.19% of moderately abnormal EEGs misclassed as severely abnormal, and 2.8% of severely abnormal or inactive misclassified as normal or mild.

The results confirm previous findings suggesting that automated classification of neonatal EEG can be achieved with good levels of accuracy. A grading system based on features extracted from quadratic time-frequency decompositions of EEG segments reported a classification accuracy of 78%, which could be augmented by incorporating supplementary information about sleep states (Stevenson et al., 2013). Improved classification performances (accuracy of 83%) were obtained by including a large feature set with a combination of probabilistic models (Gaussian Mixture Models) and supervised machine learning algorithms (Support Vector Machines, SVM), followed by additional prost-processing (Ahmed et al., 2016). Similarly, in (Moghadam et al., 2021) the combination of a large feature set with different classifiers (SVMs and neural networks) yielded very good classification performances with accuracy levels larger than 90%. More complex grading systems based on deep learning methods have been shown to provide very good accuracy levels (>85%), when applied to different EEG-based features (Raurale S, et al., 2021; Yu et al., 2023).

It is important to notice that the studies mentioned above were conducted on datasets with four HIE groups. The findings are, however, also consistent with previous classification results on three grades (mild, moderate and severe). In (Matic et al., 2014) the three groups of increasing HIE severity could be correctly identified with an accuracy of 89%. Such results were obtained by using a complex system that integrated spatial EEG information as the feature set for supervised classification methods (Matic et al., 2014). With a much simpler and faster method, a direct matching of 81% and 77% was obtained, in the initial dataset and the public EEG database, respectively.

The correct discrimination of adjacent grades (e.g. mild-moderate, moderate-severe, or moderate-severely abnormal) has been found to be a challenging task (Matic et al., 2014; Ahmed et al., 2016). In the database used, only one EEG mild graded (5.8%) was identified as moderate and vice versa. When applied to the public dataset with four grades, the approach mismatched 9.5% of moderately abnormal as severely abnormal. For the discrimination of the mild-normal HIE group from those infants requiring hypothermia, the method yielded only one misclassification (5.8%) in the dataset used, and only 13% in the Cork's data. Clinical trials have shown that therapeutic hypothermia is most beneficial for neonates with moderate grade of HIE.

Whereas the detection of normal or mild EEG is important to confirm a good clinical outcome, an accurate detection of moderate HIE cases is crucial for the identification of neonates suitable for TH. The results also showed a very good accuracy for identifying the normal or mild encephalopathy. A match in 94% of mild graded in the initial database was obtained, whereas for the Cork's data the accuracy was of 88.7% and 57% for the normal and moderately abnormal, respectively.

Although other statistical distances such as Kullback-Leibler, Hellinger or Bhattacharyya (Basseville, 1989) could be used to compare the probability distributions, the discrimination obtained from the simple variational distance proved to be better than those obtained from other alternatives. The increase of accuracy when using the Bhattacharyya distance in the Cork's data was of less than 3%.

In contrast to complex feature sets or classification algorithms, the proposed system using only a single spectral feature and a very simple classifier, yields comparable classification performances. The clinical interpretation of EEG features is of paramount importance for the development of informative biomarkers of neurological injury in infants with HIE. It is believed that the proposed 2D representation of neonatal EEG background constitutes a useful clinical decision support tool as it visually provides a traceable clue to neonathologists for the identification of full-term neonates with HIE candidates to a therapeutic hypothermia; without the black box effect of more complicated deep learning-based models (Raurale et al., 2021; Moghadam et al., 2022), or undisclosed algorithms used for automated grading systems (Hathi et al., 2010).

An accurate identification of neonates with HIE requiring a therapeutic hypothermia is of great significance to optimizes outcomes. The results show that the spectral fluctuations of EEG background, captured by the 2D level-duration distributions of delta power, can reliably be used to identify full-term neonates with HIE candidates to a therapeutic hypothermia. The system accurately identified all EEGs that should be proposed for hypothermia (moderate and severe categories), and all mild EEGs are correctly identified, except for one EEG, classified as moderate.

While these findings are encouraging, it may be further strengthened. The HIE severity was retrospectively assessed in a dataset of limited sample size. Future studies on larger patient series with prospective HIE grading can be used to further validate the approach. Furthermore, during long-term monitoring, EEG grade can change because the evolving nature of HIE. A clinical decision-making tool can integrate this dynamical behavior of HIE grades for obtaining a more flexible automated grading system (Raurale et al., 2021). Finally, the results were obtained on preselected data where EEG epoch with major artefacts were excluded. A quality index of the EEG recording can also be obtained with the inclusion of more complex artefact detection methods (Webb et al., 2021; O'Sullivan et al., 2023) to provide a more robust and informative decision support system. To expand the clinical implications of the findings, it may be evaluated on larger datasets without preselecting clean EEG segments.

The proposed approach is based on the information recorded at pre-frontal regions (or frontal electrodes for the Cork's data). It is noticed, however, that HIE discriminating power can be improved by combining combine information from multiple EEG channels (Stevenson et al., 2013; Matic et al., 2014; Yu et al., 2023), or by building more complex models that account for spatio-temporal organisation of EEGs (Garvey et al., 2021; Syvälahti et al., 2023). Nevertheless, such systems have only limited clinical utility because the newborn EEG is typically recorded at NICU with only a few channels, many of which might need rejection because of various artifacts. More sophisticated and accurate clinical decision support system could also be obtained by integrating clinical markers (Mooney et al., 2021). Similarly, functional near-infrared spectroscopy signals (Tang et al., 2024), or from other physiological variables such as heart rate variability (Pavel et al., 2023) would be interesting to develop (Chock et al., 2023).

Although much larger cohorts of patients are needed to map out the limits of the approach, the results indicated that such method can provide a robust and simple automated tool for the assessment of brain injuries in neonates with HIE. Results suggest that with the proposed system, the nursing and clinical staff could decide, based on objective arguments of the presence or absence of brain injury, whether to place a child in hypothermia. This easy-to-use decision support tool would also make it possible to reduce the delays between birth and the start of treatment when it is necessary. Above all, it will avoid imposing unjustified heavy treatment on a newborn and will allow many newborns to benefit from hypothermia and therefore limit or even eliminate future neurological disability. Deployed in a NICU, the proposed system is a could provide a real hope of reducing the number of children or adults with disabilities.

**REFERENCES**

Ahmed R, et al. Grading hypoxic-ischemic encephalopathy severity in neonatal EEG using GMM supervectors and the support vector machine. Clinical Neurophysiology (2016); 127(1): 297-309.

Antonucci R, Porcell A, Pilloni MD. Perinatal asphyxia in the term newborn. Journal of Pediatric and Neonatal Individualized Medicine (JPNIM) 2014; 3(2): e030269-e030269.

Azzopardi D, Wyatt J, Cady E et al. Prognosis of newborn infants with hypoxic-ischemic brain injury assessed by phosphorus magnetic resonance spectroscopy. Paedriatr Res 1989; 25: 445-451.

Basseville M. Distance measures for signal processing and pattern recognition. Signal Processing 1989; 18 (4): 349-369.

Boylan G, Murray D, Rennie JM. The normal EEG and aEEG. In: Rennie JM, Hagmann CF, Robertson NJ, Editors. Neonatal cerebral investigation. Cambridge University Press; 2008:83-91.

Castro Conde JR, et al. Visual and quantitative electroecephalographic analysis in healthy term neonates within the first six hours and the third day of life. Pediatric Neurology 2017; 77: 54-60.e1

Chevallier M, et al. Adherence to hypothermia guidelines: a French multicenterstudy of fullterm neonates. PLoS ONE 2013; 8(12): e83742.

Chock VY, Rao A, & Van Meurs KP. Optimal neuromonitoring techniques in neonates with hypoxic ischemic encephalopathy. Frontiers in Pediatrics (2023); 11: 1138062.

Colbourne F, et al. Prolonged but delayed postischemic hypothermia : a long-term outcome study in the rat middle cerebral artery occlusion model. J Cereb Blood Flow Metab. 2000; 20(12): 1702-1708.

Davidson J, et al. Therapeutic hypothermia for neonatal hypoxic-ischemic encephalopathy-where to from here? Frontiers in Neurology. 2015; 6: 198.

Dilena R, et al. Consensus protocol for EEG and amplitude-integrated EEG assessment and monitoring in neonates. Clinical Neurophysiology (2021); 132(4): 886-903.

Dixon G, et al. Early developmental outcomes after newborn encephalopathy. Pediatrics. 2002; 109: 26-33.

Dixon K, Smith S. In neonates with hypoxic-ischemic encephalopathy, is therapeutic hypothermia outside of current criteria safe? A literature review. Journal of Neonatal Nursing, 2019;25: 54-60.

Evans E, et al. Accuracy of amplitude integrated EEG in a neonatal cohort. Arch Dis Child Fetal Neonatal Ed 2010; 95: 169-173.

Finn D, et al. EEG for the assessment of neurological function in newborn infants immediately after birth. Arch Dis Child Fetal Neonatal. 2018; 104(5): F510-F514.

Garvey AA, et al. Multichannel EEG abnormalities during the first 6 hours in infants with mild hypoxic-ischaemic encephalopathy. Pediatric research 2021; 90(1): 117-124.

Gray P, et al. Peri-natal hypoxic-ischaemic brain injury: prediction of outcome. Dev Med Child Neurol. 1993; 35: 965-973.

Govindan RB, et al. Does relative or absolute EEG power have prognostic value in HIE setting?. Clinical Neurophysiology 2017; 128(1): 14.

Hathi M, et al. Quantitative EEG in babies at risk for hypoxic ischemic encephalopathy after perinatal asphyxia. Journal of Perinatology (2010); 30(2): 122-126.

Hellstrom-Westas L, et al. Amplitude-integrated EEG classification and interpretation in preterm and term infants. NeoReviews (2006); 7(2): e76-e87.

Jacobs S, et al. Cooling for newborns with hypoxic ischaemic encephalopathy. Cochrane Database Sys Rev. 2013; (1): CD003311

Jannatdoost A, et al. Therapeutic hypothermia head cooling and its adverse effects in Newborns with perinatal asphyxia. Intl Res J Appl Basic Sci. 2013 ;5 (12) :1546-1551

Korotchikova I, et al. EEG in the healthy term newborn within 12 hours of birth. Clinical Neurophysiology. 2009; 120 :1046-1052.

Korotchikova I, et al. Quantitative EEG analysis in neonatal hypoxic ischaemic encephalopathy. Clinical Neurophysiology (2011); 122(8): 1671-1678.

Kota S, et al. Prognostic value of continuous electroencephalogram delta power in neonates with hypoxic-ischemic encephalopathy. J Child Neurol. 2020; 35(8): 517-525.

Kota S, et al. EEG spectral power: A proposed physiological biomarker to classify the hypoxic-ischemic encephalopathy severity in real time. Pediatric Neurology 2021; 122: 7-14.

Lacan L, et al. Quantitative approach to early neonatal EEG visual analysis in hypoxic-ischemic encephalopathy severity: Bridging the gap between eyes and machine. Neurophysiologie Clinique (2021); 51(2): 121-131.

Lawn JE, Cousens S, Zupan V. 4 million neonatal deaths: when? where? Why? Lancet. 2005; 365: 891-900.

Löfhede J, et al. Automatic classification of background EEG activity in healthy and sick neonates. Journal of Neural Engineering (2010); 7(1): 016007.

Löfhede J, et al. Classification of burst and suppression in the neonatal electroencephalogram. Journal of Neural Engineering (2008); 5(4): 402.

Löfgren N, et al. Spectral distance for ARMA models applied to electroencephalogram for early detection of hypoxia. Journal of Neural Engineering (2006); 3(3): 227.

Marics G, et al. Prevalence and etiology of false normal aEEG recordings in neonatal hypoxic-ischaemic encephalopathy. BMC pediatrics (2013); 13: 1-6.

Martinello K, et al. Management and investigation of neonatal encephalopathy: 2017 update. Arch Dis Child Fetal Neonatal. 2017; 102(4): F346-F358.

Matic V, et al. Holistic approach for automated background EEG assessment in asphyxiated full-term infants. Journal of Neural Engineering (2014); 11(6): 066007.

Matic V, et al. Objective differentiation of neonatal EEG background grades using detrended fluctuation analysis. Frontiers in Human Neuroscience (2015); 9: 189.

Moghadam SM, et al. An automated bedside measure for monitoring neonatal cortical activity: a supervised deep learning-based electroencephalogram classifier with external cohort validation. The Lancet Digital Health (2022); 4(12): e884-e892.

Mooney C, et al. Predictive modelling of hypoxic ischaemic encephalopathy risk following perinatal asphyxia. Heliyon 2021; 7(7): e07411.

al Naqeeb N, et al. Assessment of neonatal encephalopathy by amplitude-integrated electroencephalography. Pediatrics (1999); 103(6): 1263-1271.

O'Sullivan ME, et al. Development of an EEG artefact detection algorithm and its application in grading neonatal hypoxic-ischemic encephalopathy. Expert Systems with Applications (2023); 213: 118917.

O'Toole JM, et al. Neonatal EEG graded for severity of background abnormalities in hypoxic-ischaemic encephalopathy. Scientific Data 2023; 10(1): 129.

Pavel AM, et al. Heart rate variability analysis for the prediction of EEG grade in infants with hypoxic ischaemic encephalopathy within the first 12 h of birth. Frontiers in pediatrics 2023; 10: 1016211.

Raurale S, et al. Grading hypoxic-ischemic encephalopathy in neonatal EEG with convolutional neural networks and quadratic time-frequency distributions. J Neural Eng March 2021; 18(4): 046007.

Roth SC, et al. Relation of deranged neonatal cerebral oxidative metabolism with neurodevelopmental outcome and head circumference at 4 years. Dev Med Child Neurol. 1997; 39(11): 718-725.

Roth SC, et al. Relation between cerebral oxidative metabolism following birth asphyxia, and neurodevelopmental outcome and brain growth at one year. Dev Med Child Neurol. 1999; 41(7): 436-445

Saliba E, Norbert K, Cantagrel S. Neuroprotection par hypothermie de l'encéphalopathie hypoxique-ischémique du nouveau-né à terme. Réanimation 2010 ;19 :655-664.

Sarnat HB, Sarnat MS. Neonatal encephalopathy following fetal distress: a clinical and electroencephalographic study. Arch Neurol. 1976; 33: 696-705.

Shellhaas RA, Soaita AI, Clancy RR. Sensitivity of amplitude-integrated electroencephalo-graphy for neonatal seizure detection. Pediatrics 2007; 120: 770-777.

Stevenson NJ, et al. An automated system for grading EEG abnormality in term neonates with hypoxic-ischaemic encephalopathy. Annals of Biomedical Engineering (2013); 41: 775-785.

Syvälahti T, et al. Networks of cortical activity show graded responses to perinatal asphyxia. Pediatric Research (2023): 1-9.

Tang L, et al. Altered resting-state functional connectivity in newborns with hypoxic ischemic encephalopathy assessed using high-density functional near-infrared spectroscopy. Scientific Reports (2024); 14(1): 3176.

Thordstein M, et al. Spectral analysis of burst periods in EEG from healthy and post-asphyctic full-term neonates. Clinical Neurophysiology (2004); 115(11): 2461-2466.

Thoresen M, et al. Time is brain: starting therapeutic hypothermia within three hours after birth improve motor outcome in asphyxiated newborns. Neonatology. 2013; 104: 228-233.

Tsuchida TN, et al. American clinical neurophysiology society standardized EEG terminology and categorization for the description of continuous EEG monitoring in neonates: report of the American Clinical Neurophysiology Society critical care monitoring committee. Journal of Clinical Neurophysiology 2013; 30(2): 161-173.

Volpe JJ. Neonatal encephalopathy: an inadequate term for hypoxic-ischemic encephalopathy. Annals of Neurology 2012; 72 (2): 156-166.

Wang X, et al. EEG phase-amplitude coupling to stratify encephalopathy severity in the developing brain. Computer Methods and Programs in Biomedicine (2022); 214: 106593.

Webb L, et al. Automated detection of artefacts in neonatal EEG with residual neural networks. Computer Methods and Programs in Biomedicine (2021); 208: 106194.

Yu S, et al. Neonatal hypoxic-ischemic encephalopathy grading from multi-channel EEG time-series data using a fully convolutional neural network. Technologies (2023); 11(6): 151.

**Claims**

1. A computer implemented method for processing an Electroencephalogram (EEG) signal, comprising

    a) Computing Power Spectral Density (PSD) of at least one frequency band of the EEG signal, for a multiplicity of consecutive time windows, to obtain computed PSD values
    b) Transforming the computed PSD values by applying a mathematical function to the computed PSD values to obtain transformed values
    c) Processing the values obtained in b) by data binning, by replacing each transformed value, which fall into a given interval by a value representative of that interval, to obtained binned values,
    d) Sequentially determining durations of the binned values, wherein a duration of a given binned value is the number of consecutive time windows for the given binned value,
    e) Creating a probability distribution of the durations of the binned values.

2. The method of claim 1, wherein the frequency band of the EEG signal is the selected from the delta waves, alpha waves, beta waves, theta waves frequency bands and association of these bands.

3. The method of claim 1, wherein PSD values are obtained from computation for delta waves frequencies and/or for theta waves frequencies.

4. The method of any one of claims 1 to 3, wherein the mathematical function is applying the $Log_{10}$ function to the computed PSD.

5. The method of claim 1, wherein PSD values are obtained from computation for delta waves frequencies and/or for theta waves frequencies and wherein the mathematical function is calculating, for each time window, the Theta power / Delta power ratio or a (Theta power + k) / (Delta power) ratio, wherein k is a number comprised between $10^3$ and $10^4$, and applying a $Log_{10}$ function to calculated ratio.

6. The method of any one of claims 1 to 5, wherein processing the values obtained in b) by data binning is performed by rounding the transformed values to the first decimal.

7. The method of any one of claims 1 to 6, further comprising smoothing the probability distribution using a gaussian kernel, preferably a (5,5) gaussian kernel.

8. The method of any one of claims 1 to 7, wherein the time window duration is 1 second.

9. The method of any one of claims 1 to 8, wherein the total duration of the EEG signal was at least 15-20 minutes, preferably about 30-40 minutes.

10. The method of any one of claims 1 to 9, wherein the EEG signal was obtained from frontal, parietal, central or occipital electrodes, or from a montage of these electrodes, preferably from frontal electrodes or from a frontal-temporal montage.

11. The method of any one of claims 1 to 10, further comprising graphically displaying the probability distribution of the duration of the binned values.

12. A computer-implemented method for grading hypoxic-ischemic encephalopathy (HIE) in an infant, comprising

   a. performing the method of any one of claims 1 to 11 with the EEG signal obtained from the infant, to obtain the probability distribution of the duration of binned values for the infant obtained from delta waves PSD or from the delta wave PSD / alpha wave PSD ratio, and
   b. calculating the distance of the probability distribution to at least two reference probability distributions, wherein multiple distances are calculated, wherein each reference probability distribution corresponds to a clinical HIE grade, and wherein the infant is affected to the clinical grade for which the calculated distance is the lowest.

13. The method of claim 12, wherein the distance is a distance selected from Bhattacharyya, Partial Curve Mapping, Area method, Discrete Frechet distance, Curve Length, Dynamic Time Warping, Mean absolute error, Mean squared error, Kullback-Leibler, Jensen Shannon and Euclidian distances.

14. The method of claim 12 or 13, wherein distances of the infant probability distribution to multiple reference distributions are calculated, wherein

   a. the lowest distance is the lowest calculated distance from the reference distributions for which there are no outliers
   b. In case there are outliers for all reference distributions, the lowest distance is set as being the one to the reference distribution for which there is the lowest number of outliers

   wherein an outlier is a point that is present in the infant probability distribution and that is absent in the reference distribution.

15. The method of any one of claims 12 to 14, which is performed on an EEG acquired from an infant during the first 6 hours of life.

**A.**

**B.**

# Figure 1

**Figure 2**

**Figure 3**

**Figure 4**

**EP 4 613 201 A1**

## EUROPEAN SEARCH REPORT

Application Number

EP 24 30 5357

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | CHO SUNGJUN ET AL: "A guide towards optimal detection of transient oscillatory bursts with unknown parameters", JOURNAL OF NEURAL ENGINEERING, IOP PUBLISHING, BRISTOL, GB, vol. 20, no. 4, 14 July 2023 (2023-07-14), XP020473720, ISSN: 1741-2560, DOI: 10.1088/1741-2552/ACDFFD [retrieved on 2023-07-14] | 1-4,7-11 | INV. A61B5/374 A61B5/00 |
| A | * page 2, right-hand column, paragraph 3 - page 10, right-hand column, paragraph 1 * * figures 1,2,6 * | 5,6, 12-15 | |
| Y | US 2019/209097 A1 (MARTIEN KATHERINE M [US] ET AL) 11 July 2019 (2019-07-11) | 1-4,7-11 | |
| A | * paragraph [0079] * * figure 4 * | 5,6, 12-15 | |
| Y | GUILLERMO B MORALES: "The dynamics of neural codes in biological and artificial neural networks", ARXIV.ORG, CORNELL UNIVERSITY LIBRARY, 201 OLIN LIBRARY CORNELL UNIVERSITY ITHACA, NY 14853, 20 February 2024 (2024-02-20), XP091686731, * page 135, last paragraph * | 7 | |

**TECHNICAL FIELDS SEARCHED (IPC)**

A61B

The present search report has been drawn up for all claims

| Place of search | Date of completion of the search | Examiner |
|---|---|---|
| The Hague | 9 August 2024 | Görlach, Tobias |

EPO FORM 1503 03.82 (P04C01)

## ANNEX TO THE EUROPEAN SEARCH REPORT
## ON EUROPEAN PATENT APPLICATION NO.

EP 24 30 5357

This annex lists the patent family members relating to the patent documents cited in the above-mentioned European search report.
The members are as contained in the European Patent Office EDP file on
The European Patent Office is in no way liable for these particulars which are merely given for the purpose of information.

09-08-2024

| Patent document cited in search report | Publication date | Patent family member(s) | | Publication date |
|---|---|---|---|---|
| US 2019209097 A1 | 11-07-2019 | EP | 3294124 A1 | 21-03-2018 |
| | | US | 2019209097 A1 | 11-07-2019 |
| | | WO | 2016187130 A1 | 24-11-2016 |

EPO FORM P0459

For more details about this annex : see Official Journal of the European Patent Office, No. 12/82

## REFERENCES CITED IN THE DESCRIPTION

**Non-patent literature cited in the description**

- **AHMED R et al.** Grading hypoxic-ischemic encephalopathy severity in neonatal EEG using GMM supervectors and the support vector machine. *Clinical Neurophysiology*, 2016, vol. 127 (1), 297-309

- **ANTONUCCI R** ; **PORCELL A** ; **PILLONI MD**. Perinatal asphyxia in the term newborn. *Journal of Pediatric and Neonatal Individualized Medicine (JPNIM)*, 2014, vol. 3 (2), e030269-e030269

- **AZZOPARDI D** ; **WYATT J** ; **CADY E et al.** Prognosis of newborn infants with hypoxic-ischemic brain injury assessed by phosphorus magnetic resonance spectroscopy. *Paedriatr Res*, 1989, vol. 25, 445-451

- **BASSEVILLE M**. Distance measures for signal processing and pattern recognition. *Signal Processing*, 1989, vol. 18 (4), 349-369

- The normal EEG and aEEG. **BOYLAN G** ; **MURRAY D** ; **RENNIE JM**. Neonatal cerebral investigation. Cambridge University Press, 2008, 83-91

- **CASTRO CONDE JR et al.** Visual and quantitative electroecephalographic analysis in healthy term neonates within the first six hours and the third day of life. *Pediatric Neurology*, 2017, vol. 77, 54-60.e1

- **CHEVALLIER M et al.** Adherence to hypothermia guidelines: a French multicenterstudy of fullterm neonates. *PLoS ONE*, 2013, vol. 8 (12), e83742

- **CHOCK VY** ; **RAO A** ; **VAN MEURS KP**. Optimal neuromonitoring techniques in neonates with hypoxic ischemic encephalopathy. *Frontiers in Pediatrics*, 2023, vol. 11, 1138062

- **COLBOURNE F et al.** Prolonged but delayed postischemic hypothermia : a long-term outcome study in the rat middle cerebral artery occlusion model.. *J Cereb Blood Flow Metab*, 2000, vol. 20 (12), 1702-1708

- **DAVIDSON J et al.** Therapeutic hypothermia for neonatal hypoxic-ischemic encephalopathy-where to from here?. *Frontiers in Neurology*, 2015 (6), 198

- **DILENA R et al.** Consensus protocol for EEG and amplitude-integrated EEG assessment and monitoring in neonates. *Clinical Neurophysiology*, 2021, vol. 132 (4), 886-903

- **DIXON G et al.** Early developmental outcomes after newborn encephalopathy. *Pediatrics*, 2002, vol. 109, 26-33

- **DIXON K** ; **SMITH S**. In neonates with hypoxic-ischemic encephalopathy, is therapeutic hypothermia outside of current criteria safe?. *A literature review. Journal of Neonatal Nursing*, 2019, vol. 25, 54-60

- **EVANS E et al.** Accuracy of amplitude integrated EEG in a neonatal cohort. *Arch Dis Child Fetal Neonatal Ed*, 2010, vol. 95, 169-173

- **FINN D et al.** EEG for the assessment of neurological function in newborn infants immediately after birth.. *Arch Dis Child Fetal Neonatal*, 2018, vol. 104 (5), F510-F514

- **GARVEY AA et al.** Multichannel EEG abnormalities during the first 6 hours in infants with mild hypoxic-ischaemic encephalopathy.. *Pediatric research*, 2021, vol. 90 (1), 117-124

- **GRAY P et al.** Peri-natal hypoxic-ischaemic brain injury: prediction of outcome. *Dev Med Child Neurol*, 1993, vol. 35, 965-973

- **GOVINDAN RB et al.** Does relative or absolute EEG power have prognostic value in HIE setting?.. *Clinical Neurophysiology*, 2017, vol. 128 (1), 14

- **HATHI M et al.** Quantitative EEG in babies at risk for hypoxic ischemic encephalopathy after perinatal asphyxia. *Journal of Perinatology*, 2010, vol. 30 (2), 122-126

- **HELLSTROM-WESTAS L et al.** Amplitude-integrated EEG classification and interpretation in preterm and term infants. *NeoReviews*, 2006, vol. 7 (2), e76-e87

- **JACOBS S et al.** Cooling for newborns with hypoxic ischaemic encephalopathy. *Cochrane Database Sys Rev*, 2013 (1), CD003311

- **JANNATDOOST A et al.** Therapeutic hypothermia head cooling and its adverse effects in Newborns with perinatal asphyxia.. *Intl Res J Appl Basic Sci.*, 2013, vol. 5 (12), 1546-1551

- **KOROTCHIKOVA I et al.** EEG in the healthy term newborn within 12 hours of birth. *Clinical Neurophysiology.*, 2009, vol. 120, 1046-1052

- **KOROTCHIKOVA I et al.** Quantitative EEG analysis in neonatal hypoxic ischaemic encephalopathy. *Clinical Neurophysiology*, 2011, vol. 122 (8), 1671-1678

- **KOTA S et al.** Prognostic value of continuous electroencephalogram delta power in neonates with hypoxic-ischemic encephalopathy. *J Child Neurol*, 2020, vol. 35 (8), 517-525

- **KOTA S et al.** EEG spectral power: A proposed physiological biomarker to classify the hypoxic-ischemic encephalopathy severity in real time. *Pediatric Neurology*, 2021, vol. 122, 7-14
- **LACAN L et al.** Quantitative approach to early neonatal EEG visual analysis in hypoxic-ischemic encephalopathy severity: Bridging the gap between eyes and machine. *Neurophysiologie Clinique*, 2021, vol. 51 (2), 121-131
- **LAWN JE ; COUSENS S ; ZUPAN V**. 4 million neonatal deaths: when? where? Why?. *Lancet*, 2005, vol. 365, 891-900
- **LÖFHEDE J et al.** Automatic classification of background EEG activity in healthy and sick neonates. *Journal of Neural Engineering*, 2010, vol. 7 (1), 016007
- **LÖFHEDE J et al.** Classification of burst and suppression in the neonatal electroencephalogram. *Journal of Neural Engineering*, 2008, vol. 5 (4), 402
- **LÖFGREN N et al.** Spectral distance for ARMA models applied to electroencephalogram for early detection of hypoxia. *Journal of Neural Engineering*, 2006, vol. 3 (3), 227
- **MARICS G et al.** Prevalence and etiology of false normal aEEG recordings in neonatal hypoxic-ischaemic encephalopathy. *BMC pediatrics*, 2013, vol. 13, 1-6
- **MARTINELLO K et al.** Management and investigation of neonatal encephalopathy: 2017 update. *Arch Dis Child Fetal Neonatal.*, 2017, vol. 102 (4), F346-F358
- **MATIC V et al.** Holistic approach for automated background EEG assessment in asphyxiated full-term infants. *Journal of Neural Engineering*, 2014, vol. 11 (6), 066007
- **MATIC V et al.** Objective differentiation of neonatal EEG background grades using detrended fluctuation analysis. *Frontiers in Human Neuroscience*, 2015, vol. 9, 189
- **MOGHADAM SM et al.** An automated bedside measure for monitoring neonatal cortical activity: a supervised deep learning-based electroencephalogram classifier with external cohort validation. *The Lancet Digital Health*, 2022, vol. 4 (12), e884-e892
- **MOONEY C et al.** Predictive modelling of hypoxic ischaemic encephalopathy risk following perinatal asphyxia. *Heliyon*, 2021, vol. 7 (7), e07411
- **NAQEEB N et al.** Assessment of neonatal encephalopathy by amplitude-integrated electroencephalography. *Pediatrics*, 1999, vol. 103 (6), 1263-1271
- **O'SULLIVAN ME et al.** Development of an EEG artefact detection algorithm and its application in grading neonatal hypoxic-ischemic encephalopathy. *Expert Systems with Applications*, 2023, vol. 213, 118917
- **O'TOOLE JM et al.** Neonatal EEG graded for severity of background abnormalities in hypoxic-ischaemic encephalopathy. *Scientific Data*, 2023, vol. 10 (1), 129
- **PAVEL AM et al.** Heart rate variability analysis for the prediction of EEG grade in infants with hypoxic ischaemic encephalopathy within the first 12 h of birth. *Frontiers in pediatrics*, 2023, vol. 10, 1016211
- **RAURALE S et al.** Grading hypoxic-ischemic encephalopathy in neonatal EEG with convolutional neural networks and quadratic time-frequency distributions. *J Neural Eng*, March 2021, vol. 18 (4), 046007
- **ROTH SC et al.** Relation of deranged neonatal cerebral oxidative metabolism with neurodevelopmental outcome and head circumference at 4 years. *Dev Med Child Neurol.*, 1997, vol. 39 (11), 718-725
- **ROTH SC et al.** Relation between cerebral oxidative metabolism following birth asphyxia, and neurodevelopmental outcome and brain growth at one year.. *Dev Med Child Neurol.*, 1999, vol. 41 (7), 436-445
- **SALIBA E ; NORBERT K ; CANTAGREL S**. Neuroprotection par hypothermie de l'encéphalopathie hypoxique-ischémique du nouveau-né à terme. *Réanimation*, 2010, vol. 19, 655-664
- **SARNAT HB ; SARNAT MS**. Neonatal encephalopathy following fetal distress: a clinical and electroencephalographic study. *Arch Neurol.*, 1976, vol. 33, 696-705
- **SHELLHAAS RA ; SOAITA AL ; CLANCY RR**. Sensitivity of amplitude-integrated electroencephalography for neonatal seizure detection. *Pediatrics*, 2007, vol. 120, 770-777
- **STEVENSON NJ et al.** An automated system for grading EEG abnormality in term neonates with hypoxic-ischaemic encephalopathy. *Annals of Biomedical Engineering*, 2013, vol. 41, 775-785
- **SYVÄLAHTI T et al.** Networks of cortical activity show graded responses to perinatal asphyxia. *Pediatric Research*, 2023, 1-9
- **TANG L et al.** Altered resting-state functional connectivity in newborns with hypoxic ischemic encephalopathy assessed using high-density functional near-infrared spectroscopy. *Scientific Reports*, 2024, vol. 14 (1), 3176
- **THORDSTEIN M et al.** Spectral analysis of burst periods in EEG from healthy and post-asphyctic full-term neonates. *Clinical Neurophysiology*, 2004, vol. 115 (11), 2461-2466
- **THORESEN M et al.** Time is brain: starting therapeutic hypothermia within three hours after birth improve motor outcome in asphyxiated newborns. *Neonatology*, 2013, vol. 104, 228-233

- **TSUCHIDA TN et al.** American clinical neurophysiology society standardized EEG terminology and categorization for the description of continuous EEG monitoring in neonates: report of the American Clinical Neurophysiology Society critical care monitoring committee. *Journal of Clinical Neurophysiology*, 2013, vol. 30 (2), 161-173
- **VOLPE JJ.** Neonatal encephalopathy: an inadequate term for hypoxic-ischemic encephalopathy. *Annals of Neurology*, 2012, vol. 72 (2), 156-166
- **WANG X et al.** EEG phase-amplitude coupling to stratify encephalopathy severity in the developing brain. *Computer Methods and Programs in Biomedicine*, 2022, vol. 214, 106593
- **WEBB L et al.** Automated detection of artefacts in neonatal EEG with residual neural networks. *Computer Methods and Programs in Biomedicine*, 2021, vol. 208, 106194
- **YU S et al.** Neonatal hypoxic-ischemic encephalopathy grading from multi-channel EEG time-series data using a fully convolutional neural network. *Technologies*, 2023, vol. 11 (6), 151